# EUROPÄISCHE PATENTANMELDUNG

(11) **EP 2 123 235 A1**
(43) Veröffentlichungstag der Anmeldung: **25.11.2009**
(21) Anmeldenummer: 08009220.8
(22) Anmeldetag: 20.05.2008
(51) Int. Cl.: A61C 8/00, A61C 13/00

(54) **Verfahren zur Herstellung eines Implantatpfostens**

(71) Anmelder: Walther, Gerd Axel, Dr., 71034 Böblingen (DE)
(72) Erfinder: Walther, Gerd Axel, Dr., 71034 Böblingen (DE)
(74) Vertreter: Vetter, Hans

(57) **Zusammenfassung**

Es wird ein Verfahren mit den folgenden Verfahrensschritten vorgeschlagen:
a) Scannen der Position wenigstens eines im Kieferknochen (10) eingesetzten Implantatkörpers;
b) Berechnen einer optimalen Geometrie des aus dem Implantatkörper (13,14) herausragenden Tragabschnitts (21,21') eines jeweiligen Implantatpfostens (17,18) aus den Positionsdaten;
c) Bearbeiten wenigstens eines Implantatpfosten-Rohlings (19) zur Erzielung der berechneten optimalen Geometrie des wenigstens einen Implantatpfostens (17,18).

## Beschreibung

Die Erfindung betrifft ein Verfahren zum Verankern eines Zahnersatzes mittels wenigstens eines Implantats in einem menschlichen Kieferknochen.

Aus der EP 0216031 A1 ist ein Implantat bekannt, dessen Implantatkörper nach Anbringen einer entsprechenden Bohrung im Kiefer verankert wird. Nach einer gewissen Ausheilungszeit wird dann ein Implantatpfosten in einer entsprechenden Ausnehmung des Implantatkörpers verankert, wobei ein aus dem Implantatkörper herausragender Tragabschnitt des Implantatpfostens zur Aufnahme und als Halterung für den Zahnersatz dient. In der Praxis können nicht zuletzt durch Unregelmäßigkeiten des Kieferknochens bedingt häufig die Implantatkörper nicht an der gewünschten idealen Stelle und in der gewünschten idealen Ausrichtung in den Kieferknochen eingesetzt werden, das heißt, sie sind entweder zu tief, zu hoch oder zu schräg positioniert. Um dies auszugleichen, sind bei der bekannten Anordnung Implantatpfosten mit unterschiedlichen Kippwinkeln zwischen Halteabschnitt und Tragabschnitt vorgesehen. Hierdurch kann eine schräge Position des Implantatkörpers im Kieferknochen bis zu einem gewissen Maße ausgeglichen werden. Es ist jedoch nicht möglich, Implantatpfosten für jeden Kippwinkel und in beliebig variierter Länge bereitzuhalten. Auch durch zeitlich aufwendiges und umständliches Ausprobieren könnte selbst dann kaum der völlig korrekte Sitz des Implantatpfostens sowie eine ausreichende Tragkraft der Aufbauten erreicht werden. Insbesondere bei solchem Zahnersatz, der aus mehreren, brückenartig miteinander verbundenen Einzelzähnen besteht und der auf mehreren Implantaten verankert wird, ist eine unterschiedliche Position und Höhe der Implantatpfosten sehr nachteilig für einen exakten Sitz und eine optimale Befestigung des Zahnersatzes. Z.B. sind auch präfabrizierte Implantataufbaupfosten bei mehrteiligen Implantatsystemen dann ungeeignet, wenn eine Keramikgerüstkonstruktion zum Befestigen auf den Aufbaupfosten angefertigt werden soll, bei der unbedingt bestimmte Mindestwandstärken in allen 3 Dimensionen aus statischen Gründen einzuhalten sind. Der behandelnde Arzt kann andererseits häufig mit seinen ihm zur Verfügung stehenden Schleifwerkzeugen diese Teile formgebend nicht bearbeiten, ohne die Keramik irreversibel in für ihn dabei nicht erkennbarer Weise zu schädigen.

Diese Art der Keramik (z.B. Zirkon) kann ausschließlich industriell hergestellt und bearbeitet werden. Der Arzt setzt nach der Herstellung nur nach Anleitung die fertigen Teile ein und kann allenfalls an der zum Schluss aufzubringenden Aufbrennkeramikschicht gewisse Korrekturen vornehmen. Aufgrund der spröden Materialeigenschaften von Keramik müssen somit in jedem Einzelfall individuelle Aufbauteile sowie die darüber zu befestigenden Supragerüstkonstruktionen (Einzelzähne oder Brücken) so gestaltet werden, dass optimale Schichtdicken, Konuswinkel und nicht zuletzt eine gemeinsame Einschubrichtung gewährleistet sind. Hierzu müssen für jede Brücken- oder Einzelzahnkonstruktion individuell die optimalen Aufbauteile, die zwischen verschiedenen Extremvarianten bei gegebenen Platzverhältnissen in einer Zahnlücke möglich sind, auch unter Berücksichtigung der Zähne des Gegenkiefers, berechnet und anschließend angefertigt werden.

Eine Aufgabe der vorliegenden Erfindung besteht darin, ein Verfahren zum Verankern eines Zahnersatzes mittels wenigstens eines Implantats aufzuzeigen, durch das auch bei nicht ideal positionierten Implantatkörpern im Kieferknochen eine einfache, sichere und exakte Positionierung des Zahnersatzes sowie eine ausreichende gute Tragkraft der Aufbauten ermöglicht wird.

Diese Aufgabe wird erfindungsgemäß durch ein Verfahren mit den Merkmalen des Anspruchs 1 gelöst.

Das Scannen der Position des wenigstens einen im Kieferknochen eingesetzten Implantats ermöglicht die Berechnung einer optimalen Geometrie des aus dem Implantatkörper herausragenden Tragabschnitts. Anhand dieser Berechnung kann aus einem Implantatpfosten-Rohling ein individuell angepasster Implantatpfosten geschaffen werden, durch den Schrägwinkel des eingesetzten Implantatkörpers und Höhenunterschiede auf einfache Weise und individuell ausgeglichen werden können. Der aus dem Implantatkörper herausragende Tragabschnitt des Implantatpfostens schafft somit wenigstens in Bezug auf Winkel und Höhe definierte Verhältnisse, so dass ein Aufsetzen des Zahnersatzes einfach und exakt möglich ist. Dies ist insbesondere bei Implantatkörpern und Implantatpfosten aus Keramikmaterial von besonderem Vorteil.

Durch die in den Unteransprüchen aufgeführten Maßnahmen sind vorteilhafte Weiterbildungen und Verbesserungen des im Anspruch 1 angegebenen Verfahrens möglich.

Zur Berechnung der Geometrie des Tragabschnitts des jeweiligen Implantatpfostens liegen in vorteilhafter Weise im Rechner bereits die Geometriedaten des Implantatkörpers selbst und/oder seiner Ausnehmung vor. Der Scan-Vorgang lässt sich somit durch entsprechende Zuordnung besonders einfach realisieren.

Die Bearbeitung des Implantatpfosten-Rohlings erfolgt bevorzugt automatisch mittels einer computergesteuerten Bearbeitungsmaschine anhand der berechneten Geometrie. Die Bearbeitung erfolgt dabei zweckmäßigerweise durch Schleifen, Fräsen oder Laser.

In vorteilhafter Weise wird lediglich der später aus dem Implantatkörper herausragende Tragabschnitt des Implantatpfosten-Rohlings bearbeitet, da sein zur Aufnahme in der Ausnehmung des Implantatkörpers vorgesehener Halteabschnitt bereits die der Ausnehmung entsprechende Gestalt besitzt, also vorfabriziert ist.

Die Verankerung des Implantatpfostens im Implantatkörper erfolgt zweckmäßigerweise in an sich bekannter Weise durch Kleben, Verklemmen, Verrasten, Bajonettverbindung und/oder Verschrauben.

Da die Position des jeweiligen Implantatkörpers und die Geometrie des Implantatpfostens bereits im Rechner gespeichert sind, liegt auch bereits die Gestalt und Position des Tragabschnitts in Form von Daten vor. Dies ermöglicht in vorteilhafter Weise die Berechnung der Geometrie der wenigstens einen Ausnehmung im Zahnersatz zur Aufnahme des Tragabschnitts des Implantatkörpers und die automatische Ausformung der Ausnehmung entsprechend dem Herstellungsverfahren für den Implantatpfosten.

Der Implantatkörper und/oder der Implantatpfosten werden bevorzugt aus einem Keramikmaterial hergestellt, sie können jedoch auch aus Metall, aus Kunststoff oder einem Karbonmaterial bestehen.

Drei Figuren sind zur Erläuterung eines Ausführungsbeispiels des Verfahrens dargestellt und in der nachfolgenden Beschreibung näher erläutert. Es zeigen:
- Figur 1: einen Abschnitt eines Kieferknochens mit zwei eingesetzten Implantatkörpern in einer Lücke zwischen zwei noch vorhandenen natürlichen Zähnen,
- Figur 2: einen Implantatpfosten-Rohling und
- Figur 3: die in Figur 1 gezeigte Darstellung bei eingesetzten Implantatpfosten und darauf aufgesetztem Zahnersatz.

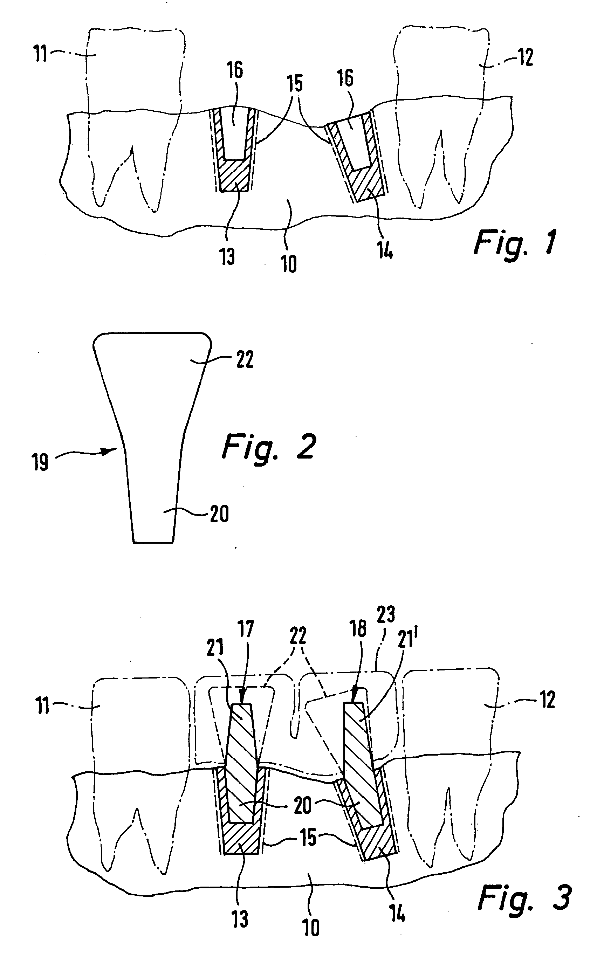

In Figur 1 ist ein Teilbereich eines menschlichen Kieferknochens 10 dargestellt, in den zwei strichpunktiert dargestellte natürliche Zähne 11, 12 eingebettet sind, zwischen denen sich eine Zahnlücke in der Breite von zwei Zähnen befindet. Um einen Zahnersatz mit einer Breite von zwei Zähnen zwischen diesen Zähnen 11, 12 fixieren zu können, sind zwei Implantatkörper 13, 14 zum Aufbau von zwei Implantaten in den Kieferknochen 10 eingesetzt. Dies erfolgt üblicherweise so, dass zunächst entsprechende Bohrungen eingebracht werden, in die dann die Implantatkörper 13, 14 eingesetzt und verankert werden. Beim Ausführungsbeispiel weisen die Implantatkörper 13, 14 eine leicht konische Außenkontur auf, die mit einem Gewinde 15 zum Einschrauben in die Bohrungen versehen sind.

Ein Kieferknochen 10 kann sehr unregelmäßig geformt sein, nicht zuletzt altersbedingt. Es ist daher nicht immer möglich, die Implantatkörper 13, 14 an der für den Zahnersatz günstigsten Stelle einzubringen. Die günstigste Stelle kann beispielsweise zu schmal, zu porös oder zu tief sein. Die Implantatkörper 13, 14 müssen daher oft neben der günstigsten Stelle positioniert werden, manchmal auch im schrägen Winkel, wie dies im Implantatkörper 14 der Fall ist.

Die Implantatkörper 13, 14 besitzen eine ebenfalls leicht konisch geformte Ausnehmung 16 zur Aufnahme von Implantatpfosten, die den Zahnersatz tragen sollen. Es handelt sich dabei um zweiteilige Implantate.

Selbstverständlich kann die Außenkontur der Implantatkörper 13, 14 beziehungsweise der Ausnehmungen 16 auch von der konischen Gestalt abweichen und beispielsweise zylindrisch sein oder eine andere Gestalt aufweisen. Auch auf ein Gewinde kann im Einzelfall verzichtet werden, wobei dann andere Fixierungsmittel häufig vorgesehen sind.

Nach dem Fixieren der Implantatkörper 13, 14 im Kieferknochen 10 werden diese gescannt und die erfassten Positionsdaten in einem nicht dargestellten Computer abgelegt. Die Geometriedaten der Ausnehmungen 16 beziehungsweise der Implantatkörper 13, 14 sind einheitlich und als Daten bereits im Computer enthalten, was die Positionserfassung durch den Scan-Vorgang vereinfacht. Aus diesen Positionsdaten werden dann die Konturen beziehungsweise Geometrien von optimalen, in die Ausnehmungen 16 einzusetzenden Implantatpfosten 17, 18 berechnet. Diese sollten zum einen möglichst senkrecht aus dem Kieferknochen vorstehen und sich bis zur gleichen Höhe erstrecken, und zwar unabhängig von den Höhenpositionen und den Neigungswinkeln der Implantatkörper 13, 14, in die sie eingesetzt werden.

Zur Herstellung der Implantatpfosten 17, 18 werden Implantatpfosten-Rohlinge 19 verwendet. Ein solcher ist in Figur 2 dargestellt. Er besitzt einen vorgefertigten Halteabschnitt 20, der zum Einsetzen in eine Ausnehmung 16 eines Implantatkörpers 13, 14 exakt dimensioniert ist. Entsprechend der Gestalt der Ausnehmung 16 besitzt er eine leicht konische Geometrie. Von diesem Halteabschnitt 20 aus erstreckt sich ein zur Ausbildung eines Tragabschnitts 21, 21' vorgesehener Bearbeitungsabschnitt 22. Dieser ist so ausgebildet, dass er eine maximale Höhe und einen maximalen Neigungswinkel des Tragabschnitts 21, 21' realisieren kann.

Anhand der berechneten Geometriedaten für den Tragabschnitt 21, 21' erfolgt nun in einer Bearbeitungsmaschine eine automatische Bearbeitung des Implantatpfosten-Rohlings 19. Diese Bearbeitung erfolgt üblicherweise durch Schleifen, insbesondere dann, wenn der Implantatpfosten-Rohling 19 aus einem Keramikmaterial besteht. Je nach Material ist jedoch auch eine Fräsbearbeitung oder eine Laserbearbeitung möglich.

Die fertigen Implantatpfosten 17, 18 werden nun gemäß Figur 3 in die Implantatkörper 13, 14 eingesetzt. Dies erfolgt in bekannter Weise durch Kleben, Verklemmen, Verrasten, Bajonettverbindung und/oder Verschrauben. Die dafür erforderliche Formgebung, insbesondere bei einer Rast- oder Bajonettverbindung oder Schraubverbindung, ist zur Vereinfachung nicht dargestellt und wird in bekannter Weise realisiert.

Die im Computer enthaltenen Geometriedaten und Positionsdaten für die Implantatkörper 13, 14 und die Geometriedaten für die Implantatpfosten 17, 18 können in einfacher Weise dazu ausgewertet werden, um die Positionen und Geometrien der aus dem Kieferknochen 10 herausstehenden Tragabschnitte 21, 21' der Implantatpfosten 17, 18 zu berechnen. Diese Daten können nun dazu verwendet werden, die entsprechenden Ausnehmungen in einem aufzusetzenden Zahnersatz 23 zur Aufnahme der Tragabschnitte 21, 21' automatisch mit Hilfe einer Bearbeitungsmaschine herzustellen. Der im vorliegenden Ausführungsbeispiel aus zwei Ersatzzähnen bestehende brückenartige Zahnersatz 23 wird dann auf die Tragabschnitte 21, 21' der Implantatpfosten 17, 18 aufgesetzt und fixiert, beispielsweise durch Verkleben und/oder Verklemmen. Hierzu besitzen die Tragabschnitte 21, 21' ebenfalls eine konusförmige Gestalt.

Anstelle der im Ausführungsbeispiel dargestellten, aus zwei Ersatzzähnen bestehenden Brücke als Zahnersatz 23 kann ein solcher Zahnersatz auch aus einem einzelnen Ersatzzahn bestehen, der auf einem einzelnen Implantatpfosten fixiert wird. Andererseits kann auch eine größere Brückenkonstruktion realisiert werden, die auf mehreren Implantatpfosten verankert wird.

Wie bereits ausgeführt, bestehen die Implantatkörper 13, 14 und die Implantatpfosten 17, 18 bevorzugt aus einem Keramikmaterial, insbesondere aus Zirkonoxid oder einer Zirkonoxid/Aluminiumoxid-Mischung, wobei auch eines oder beide Teile aus einem Metall, wie zum Beispiel Titan oder einem anderen nichtmetallischen Material bestehen kann, wie einem Kunststoffmaterial oder Karbonmaterial.

## Patentansprüche

1. Verfahren zum Verankern eines Zahnersatzes (23) mittels wenigstens eines Implantats in einem menschlichen Kieferknochen (10) mit den folgenden verfahrensschritten:
- a) Ausführen wenigstens einer Aufnahmebohrung im Kieferknochen (10) zum Einsetzen wenigstens eines Implantatkörpers (13, 14) eines Implantats, das seinerseits eine Ausnehmung (16) zur Aufnahme und Fixierung eines Implantatpfostens (17, 18) besitzt;
- b) Scannen der Position des wenigstens einen im Kieferknochen (10) eingesetzten Implantatkörpers;
- c) Berechnen einer optimalen Geometrie des aus dem Implantatkörper (13, 14) herausragenden Tragabschnitts (21, 21') des jeweiligen Implantatpfostens (17, 18) aus den Positionsdaten;
- d) Bearbeiten wenigstens eines Implantatpfosten-Rohlings (19) zur Erzielung der berechneten optimalen Geometrie des wenigstens einen Implantatpfostens (17, 18);
- e) Verankern des wenigstens einen bearbeiteten und fertiggestellten Implantatpfostens (17, 18) im wenigstens einen Implantatkörper (13, 14);
- f) Aufsetzen und Befestigen des Zahnersatzes (23) auf dem wenigstens einen Implantatpfosten (17, 18).

2. Verfahren nach Anspruch 1, **dadurch gekennzeichnet, dass** zur Berechnung der Geometrie des Tragabschnitts (21, 21') des Implantatpfostens (17, 18) in einem Computer bereits die Geometriedaten des Implantatkörpers (13, 14) und/oder seiner Ausnehmung (16) vorliegen.

3. Verfahren nach Anspruch 1 oder 2, **dadurch gekennzeichnet, dass** der Kippwinkel zwischen dem Tragabschnitt (21, 21') und dem Halteabschnitt (20) sowie die Höhe und die Dimensionierung des Tragabschnitts (21, 21') als optimale Geometriedaten berechnet werden.

4. Verfahren nach einem der vorhergehenden Ansprüche,
**dadurch gekennzeichnet, dass** die Bearbeitung des Implantatpfosten-Rohlings (19) automatisch mittels einer computergesteuerten Bearbeitungsmaschine anhand der berechneten Geometriedaten erfolgt.

5. Verfahren nach einem der vorhergehenden Ansprüche,
**dadurch gekennzeichnet, dass** die Bearbeitung des Implantatpfosten-Rohlings (19) durch Schleifen, Fräsen oder Laser erfolgt.

6. Verfahren nach einem der vorhergehenden Ansprüche,
**dadurch gekennzeichnet, dass** lediglich der später aus dem Implantatkörper (13, 14) herausragende Tragabschnitt (21, 21') des Implantatpfosten-Rohlings (19) bearbeitet wird, während sein zur Aufnahme in der Ausnehmung (16) des Implantatkörpers (13, 14) vorgesehener Halteabschnitt (20) bereits die der Ausnehmung (16) entsprechende vorgefertigte Gestalt besitzt.

7. Verfahren nach einem der vorhergehenden Ansprüche,
**dadurch gekennzeichnet, dass** die Verankerung des Implantatpfostens (17, 18) im Implantatkörper (13, 14) durch Kleben, Verklemmen, Verrasten, Bajonettverbindung und/oder Verschrauben erfolgt.

8. Verfahren nach einem der vorhergehenden Ansprüche,
**dadurch gekennzeichnet, dass** wenigstens eine Ausnehmung im Zahnersatz (23) zur Aufnahme des Tragabschnitts (21, 21') des Implantatpfostens (17, 18) entsprechend der Geometrie und der Position des Implantatpfostens (17, 18) berechnet und ausgeführt wird.

9. Verfahren nach einem der vorhergehenden Ansprüche,
**dadurch gekennzeichnet, dass** der Implantatkörper (13, 14) und/oder der Implantatpfosten (17, 18) aus Metall, aus Kunststoff, aus einem Karbonmaterial oder bevorzugt aus einem Keramikmaterial hergestellt wird.

## Geänderte Patentansprüche

### Geänderte Patentansprüche gemäss Regel 137(2) EPÜ.

**1.** Verfahren zum Verankern eines Zahnersatzes (23) auf wenigstens einem Implantat in einem menschlichen Kieferknochen (10) mit den folgenden Verfahrensschritten:
- a) Scannen der Position des wenigstens einen im Kieferknochen (10) eingesetzten keramischen Implantatkörpers;
- b) Berechnen einer optimalen Geometrie des im eingesetzten Zustand aus dem Implantatkörper (13, 14) herausragenden Tragabschnitts (21, 21') eines jeweiligen keramischen Implantatpfostens (17, 18) aus den Positionsdaten;
- c) Bearbeiten wenigstens eines Implantatpfosten-Rohlings (19) zur Erzielung der berechneten optimalen Geometrie des wenigstens einen Implantatpfostens (17, 18);
- d) Verankern eines konischen Halteabschnitts (20) des wenigstens einen bearbeiteten und fertiggestellten Implantatpfostens (17, 18) in einer entsprechenden konischen Ausnehmung (16) des wenigstens einen Implantatkörpers (13, 14) durch Kleben oder eine Bajonettverbindung;
- e) Berechnen und Ausführen wenigstens einer Ausnehmung im Zahnersatz (23) zur Aufnahme des Tragabschnitts (21, 21') des Implantatpfostens (17, 18) entsprechend der Geometrie und der Position des Implantatpfostens (17, 18);
- f) Aufsetzen und Befestigen des Zahnersatzes (23) auf dem wenigstens einen Implantatpfosten (17, 18).

**2.** Verfahren nach Anspruch 1, **dadurch gekennzeichnet, dass** zur Berechnung der Geometrie des Tragabschnitts (21, 21') des Implantatpfostens (17, 18) in einem Computer bereits die Geometriedaten des Implantatkörpers (13, 14) und/oder seiner Ausnehmung (16) vorliegen.

**3.** Verfahren nach Anspruch 1 oder 2, **dadurch gekennzeichnet, dass** der Kippwinkel zwischen dem Tragabschnitt (21, 21') und dem Halteabschnitt (20) sowie die Höhe und die Dimensionierung des Tragabschnitts (21, 21') als optimale Geometriedaten berechnet werden.

**4.** Verfahren nach einem der vorhergehenden Ansprüche, **dadurch gekennzeichnet, dass** die Bearbeitung des Implantatpfosten-Rohlings (19) automatisch mittels einer computergesteuerten Bearbeitungsmaschine anhand der berechneten Geometriedaten erfolgt.

**5.** Verfahren nach einem der vorhergehenden Ansprüche, **dadurch gekennzeichnet, dass** die Bearbeitung des Implantatpfosten-Rohlings (19) durch Schleifen, Fräsen oder Laser erfolgt.

**6.** Verfahren nach einem der vorhergehenden Ansprüche, **dadurch gekennzeichnet, dass** lediglich der später aus dem Implantatkörper (13, 14) herausragende Tragabschnitt (21, 21') des Implantatpfosten-Rohlings (19) bearbeitet wird, während sein zur Aufnahme in der Ausnehmung (16) des Implantatkörpers (13, 14) vorgesehener Halteabschnitt (20) bereits die der Ausnehmung (16) entsprechende vorgefertigte Gestalt besitzt.
